# EUROPEAN PATENT SPECIFICATION

(11) **EP 0 596 745 B1**
(45) Date of publication and mention of the grant of the patent: **27.08.1997**
(21) Application number: 93308853.6
(22) Date of filing: 05.11.1993
(51) Int. Cl.: A61K 7/04, A45D 31/00

(54) **Fingernail adhesive**
Klebstoff für künstliche Nägel
Colle pour ongle artificiel

(30) Priority: 06.11.1992 US 972794
(43) Date of publication of application: 11.05.1994
(73) Proprietor: THREE BOND OF AMERICA, INC., Torrance, California 90501 (US)
(72) Inventor: Kishita, Kazutaka, Rancho Palos Verdes, California 90274 (US); Itagaki, Yoshimi, Loveland, Ohio 45140 (US); Hirabayashi, Kazuhiro, Cincinnati, Ohio 45241 (US)
(74) Representative: Pendlebury, Anthony

(56) References cited:
- EP-A- 0 167 329
- WO-A-89/06096
- FR-A- 2 415 455
- US-A- 4 450 848
- US-A- 4 615 348

## Description

This invention relates to adhesives and particularly adhesives used for applying artificial fingernails.

In recent years, it has become fashionable among women to have long, perfect painted fingernails. Such nails are commonly referred to as "Princess Nails". Since it is difficult to keep one's natural nails long and perfect, there has developed the practice of bonding artificial fingernails made from plastic and the like onto the natural nails. Various adhesives have been utilized to perform the bonding, but the best is cyanoacrylate adhesive. Such cyanoacrylates are commonly referred to as super glue.

Since it is relatively expensive to replace the artificial nails and shape them and repaint them, the artificial nails tend to be left on as long as possible until they are broken or marred. As a result, since the hands are exposed to moisture from dish washing, clothes washing, etc., there has developed a problem with leaving the artificial nails in place over long periods of time. This problem that has developed is that a fungus will invade the area between the artificial nail and the natural nail and cause damage to the natural nail and cuticle.

Presently, the prior art method of handling such fungus infections is to remove the artificial nail with a solvent for the adhesive and to treat the fungus directly utilizing some commercially available fungicide. Since the artificial nail must be removed and it generally takes some significant time for the fungus to be eliminated, the appearance of the wearer's nails is substantially and significantly reduced.

Also, the mixing of additives into cyanoacrylate adhesive causes certain disadvantages and problems. In particular, it increases manufacturing costs and difficulties and has a significant detrimental effect upon the shelf life of the cyanoacrylate adhesive.

According to the present invention, there is provided a fingernail adhesive which includes less than one percent by weight halogen-nitropropane as a fungicide and a cyanoacrylate adhesive. Preferably the halogen-nitropropane is bromo-nitropropane, and the cyanoacrylate adhesive is selected from the group consisting of isopropyl, ethyl and methyl-cyanoacrylates and mixtures thereof.

The fingernail adhesive of the present invention is found to prevent or inhibit the growth of fungus, has a long shelf life and is easy to manufacture.

The fingernail adhesive of the present invention which has fungicide properties is made by mixing less than one percent of a halogen-nitropropane with a cyanoacrylate adhesive. The mixing can be performed by any manner commonly used in the art and by those skilled in the art and does not require any special mixing. Such halogen-nitropropanes include bromo-nitropropane, chloro-nitropropane and flouro-nitropropane. While any halogen-nitropropane is suitable for the present invention, the preferred halogen-nitropropane is bromo-nitropropane which is made by Augus Chemical Company under the brand name BROMOPOL. The general chemical formula for BROMOPOL is HOCH₂C(BR)(NO₂)CH₂OH and its C.A.S. Registry Number is 52-51-7.

While any cyanoacrylate adhesive is suitable for the present invention, the preferred types of cyanoacrylates are isopropyl-cyanoacrylate, ethyl-cyanoacrylate, methyl-cyanoacrylate and mixtures thereof. In addition, while the halogen-nitropropane may be utilized in an amount of less than one percent by weight, the preferred amount is 0.01 to 0.10 % by weight of the mixture.

Tests have been performed utilizing the above described fingernail adhesive and it has been found to have good adhesive properties, good fungicide properties and an acceptably long shelf life (more than a year).

## Claims

1. A fingernail adhesive comprising a mixture of less than one percent by weight of a halogen-nitropropane and a cyanoacrylate adhesive.

2. A fingernail adhesive according to Claim 1, wherein said cyanoacrylate adhesive is selected from the group consisting of isopropyl cyanoacrylate, ethyl cyanoacrylate, methyl cyanoacrylate and mixtures thereof.

3. A fingernail adhesive according to Claim 1 or 2, wherein said halogen-nitropropane is selected from the group consisting of bromo-nitropropane, chloro-nitropropane and fluoro-nitropropane.

4. A fingernail adhesive according to Claim 3, wherein the halogen-nitropropane is bromo-nitropropane.

5. A fingernail adhesive according to Claim 4, wherein said bromo-nitropropane is mixed with the cyanoacrylate adhesive in an amount of 0.01 to 0.10 % by weight.

## Patentansprüche

1. Ein Klebstoff für Fingernägel, enthaltend ein Gemisch von weniger als 1 Gewichtsprozent Halogennitropropan und von Cyanacrylat-Klebstoff.

2. Ein Klebstoff für Fingernägel nach Anspruch 1, wobei besagter Cyanacrylat-Klebstoff aus der Klasse, bestehend aus Isopropylcyanacrylat, Ethylcyanacrylat, Methylcyanacrylat und deren Gemischen, ausgewählt ist.

3. Ein Klebstoff für Fingernägel nach Anspruch 1 oder 2, wobei besagtes Halogennitropropan aus der Klasse, bestehend aus Bromnitropropan, Chlornitropropan und Fluornitropropan, ausgewählt ist.

4. Ein Klebstoff für Fingernägel nach Anspruch 3, wobei das Halogennitropropan Bromnitropropan ist.

5. Ein Klebstoff für Fingernägel nach Anspruch 4, wobei besagtes Bromnitropropan mit einem Anteil von 0,01 bis 0,1 Gewichtsprozent mit dem Cyanacrylat-Klebstoff vermischt ist.

## Revendications

1. Colle pour ongle artificiel comprenant un mélange d'un nitropropane halogène de moins de 1 % en poids et d'une colle cyanoacrylate.

2. Colle pour ongle artificiel selon la revendication 1, caractérisée en ce que ladite colle cyanoacrylate est choisie parmi le groupe comprenant le cyanoacrylate isopropylique, le cyanoacrylate ethylique, le cyanoacrylate methylique et des mélanges de ces produits.

3. Colle pour ongle artificiel selon la revendication 1 ou 2, caractérisée en ce le dit nitopropane halogène est choisi parmi le groupe comprenant le bromo-nitropropane, le chloro-nitropropane et le fluoro-nitropropane.

4. Colle pour ongle artificiel selon la revendication 3, caractérisée en ce le nitropropane halogène est le bromo-nitropropane.

5. Colle pour ongle artificiel selon la revendication 4, caractérisée en ce le dit bromo-nitropropane est mélangé, dans une proportion de 0.01 à 0.1 % en poids, avec la colle cyanoacrylate.
